Europäisches Patentamt

European Patent Office

Office européen des brevets

(11) Veröffentlichungsnummer: **0 430 200 B1**

## (12) EUROPÄISCHE PATENTSCHRIFT

(45) Veröffentlichungstag der Patentschrift: **08.06.94**

(21) Anmeldenummer: **90122730.6**

(22) Anmeldetag: **28.11.90**

Die Akte enthält technische Angaben, die nach dem Eingang der Anmeldung eingereicht wurden und die nicht in dieser Patentschrift enthalten sind.

(51) Int. Cl.⁵: **A61K 37/54**, A61K 37/02, A61K 47/18, //(A61K37/54, 37:02,31:195)

(54) **Arzneimittel zur subkutanen oder intramuskulären Applikation enthaltend Polypeptide.**

(30) Priorität: **29.11.89 DE 3939346**

(43) Veröffentlichungstag der Anmeldung:
**05.06.91 Patentblatt 91/23**

(45) Bekanntmachung des Hinweises auf die Patenterteilung:
**08.06.94 Patentblatt 94/23**

(84) Benannte Vertragsstaaten:
**AT BE CH DE DK ES FR GB GR IT LI LU NL SE**

(56) Entgegenhaltungen:
**EP-A- 0 156 169**
**EP-A- 0 303 251**
**US-A- 4 857 320**

(73) Patentinhaber: **BEHRINGWERKE Aktiengesell-schaft**
**Postfach 11 40**
**D-35001 Marburg(DE)**

(72) Erfinder: **Paques, Eric-Paul, Dr.**
**Schmiedeacker 18**
**W-3550 Marburg(DE)**

EP 0 430 200 B1

**Beschreibung**

Die Erfindung betrifft ein flüssiges Arzneimittel zur subkutanen (sc) Applikation enthaltend bestimmte Polypeptide und mindestens eine Aminosäure, wobei die Aminosäure eine Amino- oder Guanidino-Gruppe in der Seitenkette enthält, und ein Verfahren zur Herstellung eines solchen Arzneimittels.

Die Darreichungsformen von therapeutisch anzuwendenden Proteinen sind mit wenigen Ausnahmen auf die intravenöse Applikation begrenzt. Die orale Applikation von Polypeptiden ist in vielen Fällen erfolglos, da die Polypeptide im Magen-Darm-Trakt abgebaut werden. Es ist bereits mit Hilfe verschiedener Methoden, beispielsweise durch Mikroenkapsulierung in Liposomen, versucht worden, oral applizierbare Darreichungsformen von Polypeptiden zu entwickeln. Bis jetzt eignen sich jedoch diese Methoden nur für niedermolekulare Polypeptide.

Es wird daher versucht, die Polypeptide sc oder im zu verabreichen. Hierbei stellte sich jedoch heraus, daß die sc oder im Applikation im Vergleich zu der klassischen iv Applikation eine deutlich schlechtere Bioverfügbarkeit bietet oder mit lokalen Unverträglichkeitsreaktionen behaftet sein kann.

Die sc und im Applikationsformen bieten jedoch potentiell erhebliche Vorteile im Vergleich zu der iv Applikation.

Hierzu zählen insbesondere die Möglichkeit einer Selbstbehandlung für den Patienten und eine länger anhaltende Wirkung des Therapeutikums.

In WO 86/06962 und EP-A-0 292 908 werden Methoden zur sc oder im Applikation von Proteinen in Anwesenheit von Hydroxylamin oder Methylamin beschrieben. Es ist jedoch bekannt, daß diese Substanzen wegen ihrer begrenzten Verträglichkeit nur bedingt angewendet werden können.

In EP-A-0 303 251 ist eine injizierbare Pharmazeutische Zusammensetzung, bestehend aus modifiziertem tPA und einer Aminosäure oder einem Salz einer Aminosäure beschrieben. US-A-4 857 320 handelt von der Erhöhung der Löslichkeit von tPA in Wasser. Hierbei wird die Aminosäure Arginin zu einer wässrigen tPA-Lösung zugegeben. Durch Hinzufügen einer basischen Aminosäure wie Lysin oder Ornithin läßt sich die Löslichkeit von hochaufgereinigtem tPA in wässrigem Milieu erhöhen (EP-A-0 156 169).

Aufgabe der vorliegenden Erfindung war es, ein Mittel enthaltend eines der Polypeptide Erythropoetin, Hirudin oder Plazentaprotein 4 (PP4) zur Verfügung zu stellen, das sich für die sc Applikation eignet.

Diese Aufgabe wird erfindungsgemäß dadurch gelöst, daß in die polypeptid-haltige Lösung mindestens eine Aminosäuren, oder ein Salz einer Aminosäure eingebracht wird, wobei die Aminosäure eine Amino- oder Guanidino- Gruppe in der Seitenkette enthält. Vorzugsweise wird eine D- oder L-Aminosäure eingebracht.

Es hat sich überraschenderweise gezeigt, daß der Zusatz einer solchen Substanz die Bioverfügbarkeit und Verträglichkeit von sc applizierten Polypeptiden drastisch verbessert und somit deren sc oder im Applikation ermöglicht.

Für den erfindungsgemäßen Zweck geeignete Substanzen sind beispielsweise Lysin, Ornithin, Arginin, Diaminopimelinsäure, Agmatin, Kreatinin, Guanidinoessigsäure, Acetylornithin, Citrullin, Arginino-Bernsteinsäure, Tranexamsäure oder epsilon-Aminocapronsäure. Ein ihnen allen gemeinsames Merkmal ist die Anwesenheit einer basischen Gruppierung in Form einer Amino- oder Guanidinogruppe.

Als besonders geeignet hat sich eine Kombination von Arginin und Lysin, bevorzugt 0,001 bis 1 mol/l, besonders bevorzugt 0,01 bis 0,5 mol/l, erwiesen. Die Bioverfügbarkeit von sc oder im applizierten Polypeptiden wird durch einen solchen Zusatz deutlich erhöht im Vergleich zu einer Lösung, welche das Polypeptid und gegebenenfalls übliche Zusätze wie Stabilisatoren enthält.

Dieser günstige Einfluß dieser Substanzen auf die Bioverfügbarkeit und/oder Verbesserung der Verträglichkeit konnte besonders für t-PA (tissue plasminogen activator), eine t-PA-Mutante, Hirudin, Urokinase, AT III (Antithrombin III), Faktor XIII, EPO (Erythropoietin), von Willebrand-Faktor und PP4 (Plazenta-Protein 4) beobachtet werden. Die Aktivität der Proteine, die gemäß der Erfindung mit den beschriebenen Substanzen versetzt wurden, blieb auch nach Lyophilisation und Auflösen vor der Anwendung in sterilisiertem Wasser erhalten.

In einer erfindungsgemäßen Ausführungsform verfährt man so, daß man die polypeptidhaltige Lösung gegen einen Puffer, beispielsweise einen Phosphat-, Tris-Glycin-, Acetat- oder Citrat-Puffer, mit einer Konzentration von 0,001 bis 0,1 mol/l, vorzugsweise von 0,01 bis 0,05 mol/l mit einem pH-Wert von 2 bis 10, vorzugsweise von 4 bis 9, enthaltend mindestens eine Aminosäure, die eine weitere Aminogruppe oder eine Guanidinogruppe enthält, beispielsweise Lysin, Ornithin, Arginin, Diaminopimelinsäure, Agmatin, Kreatinin, Guanidinoessigsäure, Acetylornithin, Citrullin, Arginino-Bernsteinsäure, Tranexamsäure oder epsilon-Aminocapronsäure, vorzugsweise Lysin, Ornithin oder Arginin in einer Konzentration von 0,005 bis 0,5 mol/l, vorzugsweise von 0,01 bis 0,3 mol/l bei einer von Temperatur 2 bis 30°C, vorzugsweise 4 bis 15°C, dialysiert.

2

Die Leitfähigkeit der Dialyselösung beträgt vorzugsweise 1 bis 50 mSi (20°C), vorzugsweise 5 bis 30 mSi (20°C). Die Osmolarität liegt bei 10 bis 2000 mOsmol, vorzugsweise 100 bis 1000 mOsmol.

Nach Beendigung der Dialyse wird die polypeptidhaltige Lösung durch Konzentrierung oder Verdünnung auf die gewünschte Endkonzentration eingestellt. Die Lösung wird anschließend sterilfiltriert, abgefüllt und gegebenenfalls lyophilisiert.

Die erfindungsgemäßen Lösungen eignen sich für die Verwendung in der Human- und Veterinärmedizin gleichermaßen. Da ausschließlich physiologisch verträgliche Substanzen zugesetzt werden, treten bei der Applikation der erfindungsgemäßen Lösungen weder Entzündungen noch Hautirritationen oder Gefäßschädigungen auf.

Mit den erfindungsgemäßen Zubereitungsformen ist es jetzt möglich, durch sc Applikation Plasma-Konzentrationen an Polypeptiden zu erreichen, die sowohl eine Prophylaxe wie auch eine Therapie ermöglichen.

Die Beispiele erläutern die Erfindung.

**Beispiel 1**

Herstellung der Proteinlösungen für die sc Applikation

1. r-Hirudin wurde nach dem Verfahren von EP 0 316 650 hergestellt.
2. PP4 wurde nach dem Verfahren von EP 0 123 307 hergestellt.
3. r-Erythropoietin (EPO) wurde nach dem Verfahren von EP 0 123 307 hergestellt (1 mg EPO entspricht 80.000 E EPO-Antigen). Die Proteine wurden gegen folgende Puffer dialysiert (Tabelle 1).

Tabelle 1

| P** Endkonzentration | r-Hirudin 1.2* | PP4 5* | rEPO 2000E/ml |
|---|---|---|---|
| Dialyse gegen | | | |
| 1  0.05 M Tris  0.1 M NaCl | x | x | x |
| 2  idem pH 4.5 | | | |
| 3  0.2 M Arginin  0.2 M Lysin  pH 7.5 | x | x | x |

* = mg/ml; ** = Puffer

3

EP 0 430 200 B1

Nach der Dialyse werden die erhaltenen Lösungen sterilfiltriert und bei -20 °C bis vor Gebrauch eingefroren.

**Beispiel 2**

Je 1 ml der aufgetauten Lösungen aus Beispiel 1 wurden sc in Tiere (Ratten-oder Kaninchen) appliziert. Die Tiere wurden folgendermaßen behandelt:

| Prüfsubstanz | Dosis | Anzahl der Tiere | |
|---|---|---|---|
| | | Ratten | Kaninchen |
| | | pro Gruppe | |
| r-Hirudin | 10 mg/kg | 3 | |
| PP4 | 10 mg/kg | 3 | |
| EPO | 2000 E/kg | | 3 |

Nach 0, 30, 60, 120, 180, 240, 300, 360, 420 und 450 Minuten wurden Blutproben (+ Citrat) entnommen. Die Blutproben wurden unmittelbar danach zentrifugiert und das resultierende Citratplasma wurde bis vor der Bestimmung bei -20 °C eingefroren. Die Plasmakonzentration der Prüfsubstanz wurde wie folgt gemessen:

| r-Hirudin: | ELISA | (Fa. Behringwerke) |
|---|---|---|
| PP4: | ELISA | (Fa. Behringwerke) |
| r-Erythropoietin: | ELISA | (Fa. Behringwerke) |

Die ermittelten Daten wurden, wie in den Figuren 1 und 2 gezeigt, graphisch dargestellt. Anschließend wurde die Fläche unter der Kurve (AUC = area under the curve) berechnet. Dieser Wert ist ein Ausdruck der Bioverfügbarkeit und ermöglicht den Vergleich zwischen den unterschiedlichen Darreichungsformen. Die Daten zeigen, daß die Applikation der erfindungsgemäßen Lösungen, mit Ausnahme von Hirudin, eine deutlich bessere Bioverfügbarkeit bei der sc Applikation gewährleistet.

Überraschenderweise wurde gefunden, daß die bei der sc Applikation von Hirudin auftretenden Blutungen an dem Applikationsort mit der erfindungsgemäßen Darreichungsform deutlich seltener auftreten, als wenn diese Substanzen in deren üblichen Darreichungsformen appliziert werden.

| | | Anzahl der Tiere mit Blutungen sc |
|---|---|---|
| Hirudin | 0.05 M Tris 0.1 M NaCl | 2/3 |
| Hirudin | 0.2 M Arginin 0.2 M Lysin pH 7.5 | 0/3 |

**Patentansprüche**
**Patentansprüche für folgende Vertragsstaaten : AT, BE, CH, DE, DK, FR, GB IT, LI, LU, NL, SE**

1. Ein Arzneimittel zur subkutanen Verabreichung enthaltend in einer Lösung Erythropoetin, Hirudin oder das Plazenta-Protein 4 (PP4) und mindestens eine Aminosäure oder ein Salz einer Aminosäure, wobei die Aminosäure eine Amino- oder Guanidino-Gruppe in der Seitenkette enthält.

2. Verfahren zur Herstellung eines Arzneimittels zur subkutanen Verabreichung enthaltend Erythropoetin, Hirudin oder Plazenta-Protein 4 (PP4), dadurch gekennzeichnet, daß zu einer Lösung enthaltend Erythropoetin, Hirudin oder PP4 mindestens eine Aminosäure gegeben wird, die eine Amino- oder Guanidino-Gruppe in der Seitenkette enthält.

4

**Patentanspruch für folgende Vertragsstaaten : ES, GR**

1. Verfahren zur Herstellung eines Arzneimittels zur subkutanen Verabreichung enthaltend Erythropoetin, Hirudin oder Plazenta-Protein 4 (PP4), dadurch gekennzeichnet, daß zu einer Lösung enthaltend Erythropoetin, Hirudin oder PP4 mindestens eine Aminosäure gegeben wird, die eine Amino- oder Guanidino-Gruppe in der Seitenkette enthält.

**Claims**
**Claims for the following Contracting States : AT, BE, CH, DE, DK FR, GB, IT, LI, LU, NL, SE**

1. A pharmaceutical for subcutaneous administration containing, in a solution, erythropoietin, hirudin or placenta protein 4 (PP4) and at least one amino acid or a salt of an amino acid, the amino acid containing an amino or guanidino group in the side chain.

2. A process for the preparation of a pharmaceutical for subcutaneous administration, containing erythropoietin, hirudin or placenta protein 4 (PP4), which comprises adding at least one amino acid which contains an amino or guanidino group in the side chain to a solution containing erythropoietin, hirudin or PP4.

**Claim for the following Contracting States : ES, GR**

1. A process for the preparation of a pharmaceutical for subcutaneous administration, containing erythropoietin, hirudin or placenta protein 4 (PP4), which comprises adding at least one amino acid which contains an amino or guanidino group in the side chain to a solution containing erythropoietin, hirudin or PP4.

**Revendications**
**Revendications pour les Etats contractants suivants : AT, BE, CH, DE, DK, FR, GB, IT, LI, LU, NL, SE**

1. Médicament pour administration sous-cutanée, contenant, dans une solution, de l'érythropoïétine, de l'hirudine ou de la protéine placentaire 4 (PP4) et au moins un aminoacide ou un sel d'un aminoacide, l'aminoacide contenant un groupe amino ou guanidino dans la chaîne latérale.

2. Procédé pour la fabrication d'un médicament pour administration sous-cutanée, contenant de l'érythropoïétine, de l'hirudine ou de la protéine placentaire 4 (PP4), caractérisé en ce que l'on ajoute au moins un aminoacide, qui contient un groupe amino ou guanidino dans la chaîne latérale, à une solution contenant de l'érythropoïétine, de l'hirudine ou de la PP4.

**Revendication pour les Etats contractants suivants : ES, GR**

1. Procédé pour la fabrication d'un médicament pour administration sous-cutanée, contenant de l'érythropoïétine, de l'hirudine ou de la protéine placentaire 4 (PP4), caractérisé en ce que l'on ajoute au moins un aminoacide, qui contient un groupe amino ou guanidino dans la chaîne latérale, à une solution contenant de l'érythropoïétine, de l'hirudine ou de la PP4.